# EUROPEAN PATENT APPLICATION

(11) **EP 1 444 980 A1**
(43) Date of publication of application: **11.08.2004**
(21) Application number: 02783557.8
(22) Date of filing: 13.11.2002
(51) Int. Cl.: A61K 31/38

(54) **PREVENTIVE OR REMEDY FOR PRURITUS**

(30) Priority: 13.11.2001 JP 2001347253
(71) Applicant: KYOWA HAKKO KOGYO CO., LTD., Chiyoda-ku, Tokyo 100-8185 (JP)
(72) Inventor: HAYASHI, Ken-ichi, Kanagawa 248-0036 (JP); ICHIKAWA, Shunji, Sunto-gun, Shizuoka 411-8731 (JP); KARASAWA, Akira , c/o Head Office,, Chiyoda-ku, Tokyo100-8185 (JP)
(74) Representative: VOSSIUS & PARTNER
(86) International application number: PCT/JP2002/011830
(87) International publication number: WO 2003/041704

(57) **Abstract**

A preventive or therapeutic agent for pruritus which comprises, as an active ingredient, a tricyclic compound represented by Formula (I): 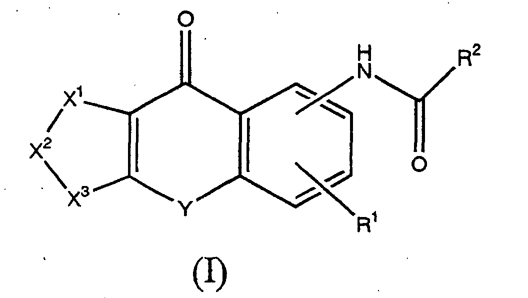 {wherein R¹ represents hydrogen, substituted or unsubstituted lower alkyl, substituted or unsubstituted lower alkoxy or halogen;
X¹-X²-X³ represents CR⁵=CR⁶-CR⁷=CR⁸ [wherein R⁵, R⁶, R⁷ and R⁸, which may be the same or different, each represent hydrogen, substituted or unsubstituted lower alkyl, hydroxy, substituted or unsubstituted lower alkoxy, nitro, amino, mono(lower alkyl)-substituted amino, di(lower alkyl)-substituted amino, substituted or unsubstituted lower alkanoylamino or halogen], N(O)ₘ=CR⁶-CR⁷=CR⁸ (wherein R⁶, R⁷ and R⁸ 'each have the same significances as defined above, and m represents 0 or 1), etc.; Y represents -CH₂S-, -CH₂SO-, -CH₂SO₂-, -CH₂O-, -CH=CH-, -(CH₂)ₚ- (wherein p represents an integer of 0 to 2), -SCH₂-, -SOCH₂-, -SO₂CH₂- or -OCH₂-; and
R² represents hydrogen, substituted or unsubstituted lower alkyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, or a substituted or unsubstituted heterocyclic group} or a pharmaceutically acceptable salt thereof.

## Description

### Technical Field

The present invention relates to a preventive or therapeutic agent for pruritus.

### Background Art

It is known that not only allergic skin disorders such as chronic urticaria and atopic dermatitis but also systemic disorders such as chronic renal insufficiency, hepatocholestasis, hematopoietic disorders, endocrine disorders and malignant tumors may be accompanied by skin pruritus [Drugs, Vol. 39, pp. 218-223 (1990)]. It'is considered that histamine may be one substance to cause pruritus. However, antihistamines are clinically effective for some types of pruritus but can not inhibit all types of pruritus.

On the other hand, tricyclic compounds and their pharmaceutically acceptable salts having the activity to prolong the intervals of bladder contractions are known as therapeutic agents for urinary incontinence (WO97/14672 and WO98/46587).

### Disclosure of the Invention

An object of the present invention is to provide a preventive or therapeutic agent for pruritus associated with allergic skin disorders or systemic disorders.

The present invention relates to the following:
(1) a preventive or therapeutic agent for pruritus which comprises, as an active ingredient, a tricyclic compound represented by Formula (I): 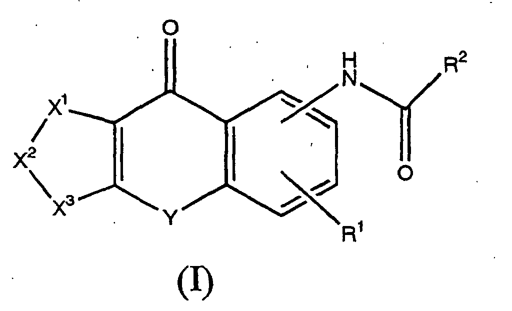 {wherein R¹ represents hydrogen, substituted or unsubstituted lower alkyl, substituted or unsubstituted lower alkoxy or halogen; X¹-X²-X³ represents CR⁵=CR⁶-CR⁷=CR⁸ [wherein R⁵, R⁶, R⁷ and R⁸, which may be the same or different, each represent hydrogen, substituted or unsubstituted lower alkyl, hydroxy, substituted or unsubstituted lower alkoxy, nitro, amino, mono(lower alkyl)-substituted amino, di(lower alkyl)-substituted amino, substituted or unsubstituted lower alkanoylamino or halogen], N(O)ₘ=CR⁶-CR⁷=CR⁸ (wherein R⁶, R⁷ and R⁸ each have the same significances as defined above, and m represents 0 or 1), CR⁵=CR⁶-N(O)ₘ=CR⁸ (wherein R⁵, R⁶, R⁸ and m each have the same significances as defined above), CR⁵=CR⁶-CR⁷=N(O)ₘ (wherein R⁵, R⁶, R⁷ and m each have the same significances as defined above), CR⁵=CR⁶-O (wherein R⁵ and R⁶ each have the same significances as defined above); CR⁵=CR⁶-S (wherein R⁵ and R⁶ each have the same significances as defined above), O-CR⁷=CR⁸ (wherein R⁷ and R⁸ each have the same significances as defined above), S-CR⁷=CR⁸ (wherein R⁷ and R⁸ each have the same significances as defined above) or O-CR⁷=N (wherein R⁷ has the same significance as defined above);
   Y represents -CH₂S-, -CH₂SO-, -CH₂SO₂-, -CH₂O-, -CH=CH-, -(CH₂)ₚ- (wherein p represents an integer of 0 to 2), -SCH₂-, -SOCH₂-, -SO₂CH₂- or -OCH₂-; and
   R² represents hydrogen, substituted or unsubstituted lower alkyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, or a substituted or unsubstituted heterocyclic group} or a pharmaceutically acceptable salt thereof,
(2) a preventive or therapeutic agent for pruritus which comprises, as an active ingredient, a tricyclic compound represented by Formula (Ia): 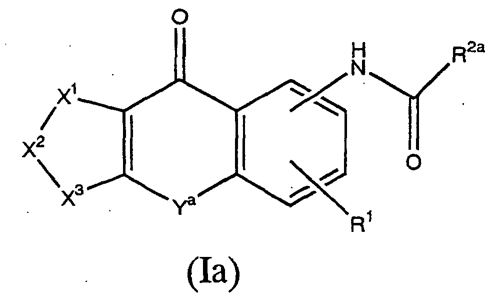 [wherein R¹ and X¹-X²-X³ each have the same significances as defined above; Y^{a} represents -CH₂SO₂-, -SCH₂-, -SOCH₂-, -SO₂CH₂- or -OCH₂-; and
   when Y^{a} is -CH₂SO₂-, -SCH₂-, -SOCH₂- or -SO₂CH₂-,
   R^{2a} represents hydrogen, substituted or unsubstituted lower alkyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, or a substituted or unsubstituted nitrogen-containing heterocyclic group; and
   when Y^{a} is -OCH₂-,
   R^{2a} represents hydrogen, trifluoromethyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, a substituted or unsubstituted nitrogen-containing heterocyclic group, or Formula (II): 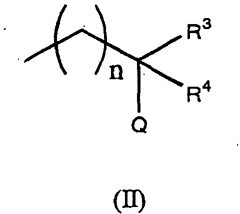 (wherein n is 0 or 1; R³ and R⁴, which may be the same or different, each represent hydrogen, substituted or unsubstituted lower alkyl, substituted or unsubstituted cycloalkyl, substituted or unsubstituted aryl, or substituted or unsubstituted aralkyl, or R³ and R⁴ may be combined together with the adjacent carbon atom to form cycloalkyl; and Q represents hydroxy, substituted or unsubstituted lower alkoxy, amino or halogen)] or a pharmaceutically acceptable salt thereof,
(3) a preventive or therapeutic agent for pruritus which comprises, as an active ingredient, the tricyclic compound or the pharmaceutically acceptable salt thereof described in (2), wherein Y^{a} is -CH₂SO₂-, -SCH₂-, -SOCH₂- or -SO₂CH₂-,
(4) a preventive or therapeutic agent for pruritus which comprises, as an active ingredient, the tricyclic compound or the pharmaceutically acceptable salt thereof described in (2), wherein Y^{a} is -OCH₂-,
(5) a preventive or therapeutic agent for pruritus which comprises, as an active ingredient, the tricyclic compound or the pharmaceutically acceptable salt thereof described in any of (2) to (4), wherein R¹ is hydrogen, substituted or unsubstituted lower alkoxy or halogen,
(6) a preventive or therapeutic agent for pruritus which comprises, as an active ingredient, the tricyclic compound or the pharmaceutically acceptable salt thereof described in any of (2) to (4), wherein R¹ is hydrogen,
(7) a preventive or therapeutic agent for pruritus which comprises, as an active ingredient, the tricyclic compound or the pharmaceutically acceptable salt thereof described in any of (2), (5) and (6), wherein Y^{a} is -CH₂SO₂-, -SO₂CH₂- or -OCH₂-,
(8) a preventive or therapeutic agent for pruritus which comprises, as an active ingredient, the tricyclic compound or the pharmaceutically acceptable salt thereof described in any of (2), (5) and (6), wherein Y^{a} is -CH₂SO₂- or -SO₂CH₂-,
(9) a preventive or therapeutic agent for pruritus which comprises, as an active ingredient, the tricyclic compound or the pharmaceutically acceptable salt thereof described in any of (2), (5) and (6), wherein Y^{a} is -CH₂SO₂-,
(10) a preventive or therapeutic agent for pruritus which comprises, as an active ingredient, the tricyclic compound or the pharmaceutically acceptable salt thereof described in any of (2) to (9), wherein X¹-X²-X³ is S-CR⁷=CR⁸ (wherein R⁷ and R⁸ each have the same significances as defined above),
(11) a preventive or therapeutic agent for pruritus which comprises, as an active ingredient, the tricyclic compound or the pharmaceutically acceptable salt thereof described in any of (2) to (9), wherein X¹-X²-X³ is CR⁵=CR⁶-CR⁷=CR⁸ (wherein R⁵, R⁶, R⁷ and R⁸ each have the same significances as defined above),
(12) a preventive or therapeutic agent for pruritus which comprises, as an active ingredient, the tricyclic compound or the pharmaceutically acceptable salt thereof described in any of (2) to (11), wherein R^{2a} is Formula (II): 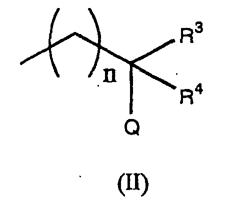 (wherein n, R³, R⁴ and Q each have the same significances as defined above),
(13) a preventive or therapeutic agent for pruritus which comprises, as an active ingredient, the tricyclic compound or the pharmaceutically acceptable salt thereof described in (12), wherein n is 0,
(14) a preventive or therapeutic agent for pruritus which comprises, as an active ingredient, the tricyclic compound or the pharmaceutically acceptable salt thereof described in (13), wherein R³ is methyl, R⁴ is trifluoromethyl, and Q is hydroxy,
(15) a preventive or therapeutic agent for pruritus which comprises, as an active ingredient, the tricyclic compound or the pharmaceutically acceptable salt thereof described in (2), wherein R¹ is hydrogen, Y^{a} is -CH₂SO₂-, X¹-X²-X³ is S-CR⁷=CR⁸ (wherein R⁷ and R⁸ each have the same significances as defined above), and R² is Formula (III): 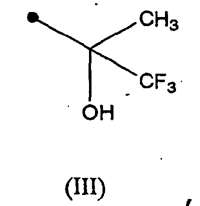
(16) a preventive or therapeutic agent for pruritus which comprises, as an active ingredient, a tricyclic compound represented by Formula (Ib): 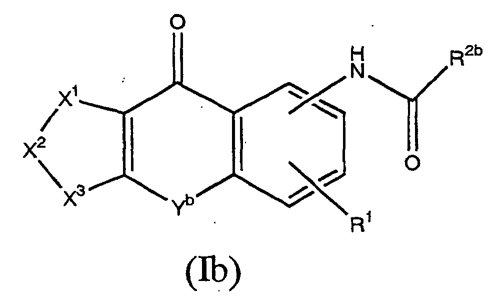 [wherein R¹ and X¹-X²-X³ each have the same significances as defined above;
   Y^{b} represents -CH₂O-, -CH₂S-, -CH₂SO-, -CH=CH- or -(CH₂)_{P}- (wherein p has the same significance as defined above); and
   R^{2b} represents Formula (III): 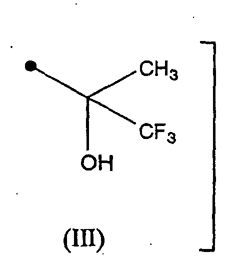 or a pharmaceutically acceptable salt thereof,
(17) a preventive or therapeutic agent for pruritus which comprises, as an active ingredient, the tricyclic compound or the pharmaceutically acceptable salt thereof described in (16), wherein X¹-X²-X³ is CR⁵=CR⁶-CR⁷=CR⁸ (wherein R⁵, R⁶, R⁷ and R⁸ each have the same significances as defined above) or CR⁵=CR⁶-CR⁷=N (wherein R⁵, R⁶ and R⁷ each have the same significances as defined above),
(18) a preventive or therapeutic agent for pruritus which comprises, as an active ingredient, the tricyclic compound or the pharmaceutically acceptable salt thereof described in (16), wherein X¹-X²-X³ is CR⁵=CR⁶-O (wherein R⁵ and R⁶ each have the same significances as defined above) or CR⁵=CR⁶-S (wherein R⁵ and R⁶ each have the same significances as defined above),
(19) a preventive or therapeutic agent for pruritus which comprises, as an active ingredient, the tricyclic compound or the pharmaceutically acceptable salt thereof described in (16), wherein X¹-X²-X³ is O-CR⁷=CR⁸ (wherein R⁷ and R⁸ each have the same significances as defined above) or S-CR⁷=CR⁸ (wherein R⁷ and R⁸ each have the same significances as defined above),
(20) a preventive or therapeutic agent for pruritus which comprises, as an active ingredient, the tricyclic compound or the pharmaceutically acceptable salt thereof described in any of (16) to (19), wherein Y^{b} is -CH₂O-,
(21) a preventive or therapeutic agent for pruritus which comprises, as an active ingredient, the tricyclic compound or the pharmaceutically acceptable salt thereof described in any of (16) to (19), wherein Y^{b} is -(CH₂)ₚ- (wherein p has the same significance as defined above),
(22) a preventive or therapeutic agent for pruritus which comprises, as an active ingredient, the tricyclic compound or the pharmaceutically acceptable salt thereof described in (21), wherein p is 0,
(23) a preventive or therapeutic agent for pruritus which comprises, as an active ingredient, the tricyclic compound or the pharmaceutically acceptable salt thereof described in (21), wherein p is 2,
(24) a preventive'or therapeutic agent for pruritus which comprises, as an active ingredient, the tricyclic compound or the pharmaceutically acceptable salt thereof described in any of (16) to (19), wherein Y^{b} is -CH=CH-,
(25) a preventive or therapeutic agent for pruritus which comprises, as an active ingredient, the tricyclic compound or the pharmaceutically acceptable salt thereof described in any of (16) to (19), wherein Y^{b} is -CH₂S- or -CH₂SO-, and
(26) use of the tricyclic compound or the pharmaceutically acceptable salt thereof described in any of (1) to (25) for the manufacture of a pharmaceutical composition useful for prevention or treatment of pruritus.
   The present invention also relates to the following:
(27) a method for preventing or treating pruritus, which comprises a step of administering the tricyclic compound or the pharmaceutically acceptable salt thereof described in any of (1) to (25).

Hereinafter, the compounds represented by Formula (I) are referred to as Compounds (I), and the same applies to the compounds of other formula numbers.

In the definitions of the groups in Formula (I), the lower alkyl includes, for example, straight-chain or branched lower alkyl groups having 1 to 8 carbon atoms, more specifically methyl, ethyl, propyl, isopropyl, butyl, isobutyl, sec-butyl, tert-butyl, pentyl, hexyl, 1,2,2-trimethylpropyl, heptyl, octyl, etc.

The halogen means a fluorine, chlorine, bromine and iodine atom.

The lower alkyl moiety of the lower alkoxy, mono(lower alkyl)-substituted amino and di(lower alkyl)-substituted amino has the same significance as that of the lower alkyl group mentioned above.

The lower alkanoyl moiety of the lower alkanoylamino includes, for example, alkanoyl groups having 1 to 6 carbon atoms , more specifically formyl, acetyl, propanoyl, butanoyl, pentanoyl, 2,2-dimethylpropanoyl, hexanoyl, etc.

The lower alkenyl includes, for example, straight-chain or branched lower alkenyl groups having 2 to 6 carbon atoms, more specifically vinyl, allyl, 1-propenyl, methacryl, 1-butenyl, crotyl, pentenyl, hexenyl, etc.

The aryl and the aryl moiety of the arylamino include, for example, phenyl, naphthyl, etc; and the heteroaryl includes, for example, pyridyl, furyl, thienyl, quinolyl, imidazolyl, benzimidazolyl, thiazolyl, etc.

The aralkyl moiety of the aralkylamino includes, for example, aralkyl groups having 7 to 12 carbon atoms, more specifically benzyl, phenethyl, naphthylmethyl, etc.

The heterocyclic group includes, for example, heteroalicyclic groups, nitrogen-containing heterocyclic groups, etc. The heteroalicyclic group includes, for example, tetrahydrofuryl, tetrahydrothienyl, chromanyl, etc. The nitrogen-containing heterocyclic group includes, for example, heterocyclic groups containing one or two nitrogen atoms in the ring and optionally containing other hetero atoms such as oxygen, sulfur, etc. For example, it includes pyrrolidinyl, pipecolinyl, piperazinyl, piperidinyl, morpholinyl, thiomorpholinyl, oxazolyl, etc.

The substituted lower alkyl, the substituted lower alkoxy, the mono(substituted lower alkyl)-substituted amino, the di (substituted lower alkyl)-substituted amino, the substituted lower alkanoylamino and the substituted lower alkenyl each have 1 to a substitutable number (preferably 1 to 6, more preferably 1 to 4) of substituents which are the same or different. Examples of the substituents include hydroxy, halogen, nitro, amino, mono(lower alkyl)-substituted amino, di(lower alkyl)-substituted amino, cycloalkyl, substituted cycloalkyl [the substituted cycloalkyl has 1 to 3 substituents which are the same or different, such as hydroxy, halogen, nitro, amino, mono(lower alkyl)-substituted amino, di(lower alkyl)-substituted amino, lower alkoxy, etc], aryl, substituted aryl (the substituent in the substituted aryl has the same significance as that in the substituted aryl described below), aralkyl, substituted aralkyl (the substituent in the substituted aralkyl has the same significance as that in the substituted aralkyl described below), lower alkoxy, substituted lower alkoxy [the substituted lower alkoxy has 1 to 3 substituents which are the same or different, such as hydroxy, halogen, nitro, amino, mono(lower alkyl)-substituted amino, di(lower alkyl)-substituted amino, lower alkoxy, etc]. Further, the two lower alkyl moieties on the same carbon atom of the substituted lower alkyl may form, together with the said carbon atom, an aliphatic ring. When the substituted lower alkyl is substituted methyl or substituted ethyl, the number of the substituents may be, for example, 1 to 3 and the substituents may be the same or different. For example, the substituent includes lower alkyl, substituted lower alkyl [the substituted lower alkyl has 1 to 3 substituents which are the same or different, such as hydroxy, halogen, nitro, amino, mono(lower alkyl)-substituted amino, di(lower alkyl)-substituted amino, lower alkoxy, etc], etc.

In the definition of the substituents of the substituted lower alkyl, substituted lower alkoxy, mono(substituted lower alkyl)-substituted amino, di(substituted lower alkyl)-substituted amino, substituted lower alkanoylamino and substituted lower alkenyl, the halogen has the same significance as defined above, the lower alkyl and the lower alkyl moiety of the mono(lower alkyl)-substituted amino, di(lower alkyl)-substituted amino and lower alkoxy have the same significance as that of the above-described lower alkyl, and the aryl has the same significance as defined above. The cycloalkyl and the cycloalkyl moiety of aliphatic rings include, for example, cycloalkyl groups having 3 to 8 carbon atoms, more specifically cyclopropyl, cyclobutyl, cyclopentyl, cyclohexyl, cycloheptyl, cyclooctyl, etc. The aralkyl includes, for example, aralkyl groups having 7 to 12 carbon atoms, more specifically benzyl, phenethyl, naphthylmethyl, etc.

The substituted aryl, the substituted heteroaryl, the substituted aralkylamino and the substituted arylamino each have 1 to 3 substituents which are the same or different. Examples of the substituents includes lower alkyl, hydroxy, amino, halogen, etc.

The lower alkyl and halogen in the definition of the substituents in the substituted aryl, substituted heteroaryl, substituted aralkylamino and substituted arylamino have the same significances as defined above, respectively.

The substituted heterocyclic group has 1 to 3 substituents which are the same or different. Examples of the substituents includes lower alkyl, hydroxy, halogen, etc.

The lower alkyl and halogen in the definition of the substituents in the substituted heterocyclic group have the same significances as defined above, respectively.

In the definitions of formula (Ia) and formula (Ib), the lower alkyl includes, for example, straight-chain or branched lower alkyl groups having 1 to 6 carbon atoms, more specifically methyl, ethyl, propyl, isopropyl, butyl, isobutyl, sec-butyl, tert-butyl, pentyl, hexyl, 1,2,2-trimethylpropyl, etc.

The halogen means a fluorine, chlorine, bromine and iodine atom.

The lower alkyl moiety of the lower alkoxy, mono(lower alkyl)-substituted amino and di(lower alkyl) -substituted amino has the same significance as that of the lower alkyl group mentioned above.

The lower alkenyl includes, for example, straight-chain or branched lower alkenyl groups having 2 to 6 carbon atoms, more specifically vinyl, allyl, 1-propenyl, methacryl, 1-butenyl, crotyl, pentenyl, hexenyl, etc.

The aryl and the aryl moiety of the arylamino include, for example, phenyl, naphthyl, etc; and the heteroaryl includes, for example, pyridyl, furyl, thienyl, quinolyl, imidazolyl, benzimidazolyl, thiazolyl, etc.

The aralkyl and the aralkyl moiety of the aralkylamino include, for example, aralkyl groups having 7 to 12 carbon atoms, more specifically benzyl, phenethyl, naphthylmethyl, etc.

The heteroalicyclic group includes, for example, tetrahydrofuryl, tetrahydrothienyl, chromanyl, etc. The nitrogen-containing heterocyclic group includes, for example, a heterocyclic group containing one or two nitrogen atoms in the ring and optionally containing other hetero atoms such as oxygen and sulfur, in which the nitrogen atom in the ring bonds to the adjacent carbonyl group. For example, it includes pyrrolidinyl, pipecolinyl, piperazinyl, piperidinyl, morpholinyl, thiomorpholinyl, oxazolyl, etc.

The cycloalkyl includes, for example, cycloalkyl groups having 3 to 8 carbon atoms, more specifically cyclopropyl, cyclobutyl, cyclopentyl, cyclohexyl, cycloheptyl, cyclooctyl, etc.

The substituted lower alkyl, the substituted lower alkoxy, the mono(substituted lower alkyl)-substituted amino, the di(substituted lower alkyl)-substituted amino, the substituted lower alkenyl and the substituted cycloalkyl each have 1 to 3 substituents which are the same or different. Examples of the substituents include hydroxy, halogen, nitro, amino, mono (lower alkyl)-substituted amino, di(lower alkyl)-substituted amino, lower alkoxy, etc. When the substituted lower alkyl is substituted methyl or substituted ethyl, the number of the substituents may be, for example, 1 to 3 and the substituents may be the same or different. For example, the substituent includes lower alkyl, substituted lower alkyl [the substituted lower alkyl has 1 to 3 substituents which are the same or different, such as hydroxy, halogen, nitro, amino, mono(lower alkyl)-substituted amino, di(lower alkyl)-substituted amino, lower alkoxy, etc], cycloalkyl, substituted cycloalkyl [the substituted cycloalkyl has 1 to 3 substituents which are the same or different, such as hydroxy, halogen, nitro, amino, mono(lower alkyl)-substituted amino, di(lower alkyl)-substituted amino, lower alkoxy, etc], aryl, substituted aryl [the substituted aryl has 1 to 3 substituents which are the same or different, such as hydroxy, halogen, nitro, amino, mono(lower alkyl)-substituted amino, di(lower alkyl)-substituted amino, lower alkoxy, etc], aralkyl, substituted aralkyl [the substituted aralkyl has 1 to 3 substituents which are the same or different, such as hydroxy, halogen, nitro, amino, mono(lower alkyl)-substituted amino, di(lower alkyl)-substituted amino, lower alkoxy, etc],' substituted lower alkoxy [the substituted lower alkoxy has 1 to 3 substituents which are the same or different, such as hydroxy, halogen, nitro, amino, mono(lower alkyl)-substituted amino, di(lower alkyl)-substituted amino, lower alkoxy, etc]. Further, the two lower alkyl groups on the same carbon atom of the substituted methyl or substituted ethyl may form, together with the said carbon atom, an aliphatic ring.

In the definition of the substituents of the substituted lower alkyl, substituted lower alkoxy, mono (substituted lower alkyl)-substituted amino, di(substituted lower alkyl)-substituted amino, substituted lower alkenyl and substituted cycloalkyl, the halogen, the cycloalkyl, the cycloalkyl moiety of the aliphatic ring, the aryl and the aralkyl have the same significances as defined above, respectively, the lower alkyl and the lower alkyl moiety of the mono(lower alkyl)-substituted amino, di(lower alkyl)-substituted amino and lower alkoxy have the same significance as that of the above-described lower alkyl.

The substituted aryl, the substituted heteroaryl, the substituted aralkyl, the substituted aralkylamino and the substituted arylamino each have 1 to 3 substituents which are the same or different. Examples of the substituents include lower alkyl, hydroxy, amino, halogen, etc.

In the definition of the substituents of the substituted aryl, substituted heteroaryl, substituted aralkyl, substituted aralkylamino and substituted arylamino, the lower alkyl and the halogen have the same significances as defined above, respectively.

The substituted heteroalicyclic group and the substituted nitrogen-containing heterocyclic group each have 1 to 3 substituents which are the same or different. Examples of the substituents include lower alkyl, hydroxy, halogen, etc.

In the definition of the substituents in the substituted heteroalicyclic group and the substituted nitrogen-containing heterocyclic group, the lower alkyl and the halogen have the same significances as defined above, respectively.

The pharmaceutically acceptable salts of Compound (I), Compound (Ia) and Compound (Ib) include pharmaceutically acceptable acid addition salts, for example, inorganic acid addition salts such as hydrochloride, hydrobromide, hydroiodide, nitrate, sulfate and phosphate, and organic acid addition salts such as formate, acetate, benzoate, maleate, fumarate, succinate, tartrate, citrate, oxalate, glyoxylate, aspartate, methanesulfonate, ethanesulfonate and benzenesulfonate.

The tricyclic compounds used in the present invention can be produced according to the methods disclosed in the above publications or similar methods, and can be isolated and purified by purification methods conventionally used in synthetic organic chemistry, such as neutralization, filtration, extraction, washing, drying, concentration, recrystallization and various kinds of chromatography.

When it is desired to obtain a salt of the tricyclic compound used in the present invention, in the case where it is produced in the form of the salt, it can be subjected to purification as such, and where it is produced in the form of a free base, it can be converted into a salt, after being dissolved or suspended in a suitable solvent, by adding an acid or a base thereto.

There may be optical isomers for some of the tricyclic compounds used in the present invention. All possible stereoisomers and mixtures thereof can be used as active ingredients of the preventive or therapeutic agent of the present invention.

The tricyclic compounds or pharmaceutically acceptable salts thereof used in the invention may exist in the form of adducts with water or various solvents, which can also be used as active ingredients of the preventive or therapeutic agent of the invention.

The pharmacological activities of typical Compound (I) are described in test examples. In Test Example 1, (S)-(+)-3,3,3-trifluoro-2-hydroxy-2-methyl-N-(5,5,10-trioxo - 4,10-dihydrothieno[3,2c][1]benzothiepin-9-yl)propanamide, and 2-hydroxy-2-methyl-N-(5,5,10-trioxo-4,10-dihydrothieno [3,2c][1]benzothiepin-9-yl)propanamide were used as test compounds. Hereinafter, the above compounds are referred to as Compound 1 and Compound 2, respectively. In Test Examples 2 and 3, Compound 1 was used as a test compound. Compound 1 and Compound 2 are the same compounds as Compound (1-25) and Compound (3-12), respectively, described in WO98/46587. Test Example 1: Inhibitory effect on compound 48/80-induced scratching in mice

The experiment was carried out according to the method of Kuraishi, et al. [*Eur. J. Pharmacol*., Vol. 275, pp. 229-233 (1995)].

Male ddY mice each weighing 19 to 22 g (supplied by Japan SLC) were used in the test. 25 to 26 mice were put in each plastic cage and reared by allowing them to freely take commercially available solid feed and water, in an animal room at 19-25°C, 30-70% humidity and a 12 h lighting cycle (lighting: 7:00 a.m. to 7:00 p.m.) a day.

0.1 ml of a solution of compound 48/80 (by Sigma) (0.1 mg of compound 48/80 was dissolved in 1 ml of physiological saline) was subcutaneously injected into the right-side back shoulder of each mouse, and all the mice were individually put in a transparent cage. For 1 hour immediately after this, the behavior of every mouse was recorded with an 8-mm video camera recorder. The video was reproduced, and the number of the scratching by its right hind pawwas counted for 1 hour immediately after the subcutaneous administration, at intervals of 5 minutes or 10 minutes, and the data of every 5 or 10 minutes were totaled. Each mouse scratched itself a few times per about one second, and a series of these actions of each mouse was counted as one.

Compound 1 and Compound 2 were separately suspended in aqueous 0.5 w/v % methyl cellulose solution to have a concentration of 3 mg/ml. The suspension of Compound 1 was further diluted with aqueous 0.5 w/v % methyl cellulose solution, and suspensions or solutions for administration each having an intended concentration were prepared. The solvent (aqueous 0.5 w/v % methyl cellulose solution) or the suspension or solution for administration was orally administered to each mouse, 30 minutes before the subcutaneous injection of the compound 48/80, and its volume was 10 ml/kg. The solvent-administered mice were a control group.

The data of Compound 1 (3-30 mg/kg, p.o.) and Compound 2 (each 30 mg/kg, p.o.) to inhibit the compound 48/80-induced scratching in mice are shown in Table 1 and Table 2.

**Table 1**

| Time after Subcutaneous Injection (min) | Control | Compound 1 (mg/kg, p.o.) | | |
|---|---|---|---|---|
| | | 3 | 10 | 30 |
| 0-5 | 5 ± 2 | 3 ± 1 | 2 ± 1 | 0 ± 0* |
| 5-10 | 11 ± 3 | 7 ± 2 | 6 ± 2 | 0 ± 0*** |
| 10-15 | 16 ± 4 | 9 ± 1 | 8 ± 2 | 1 ± 1** |
| 15-20 | 24 ± 5 | 12 ± 4 | 9 ± 2 | 2 ± 1*** |
| 20-25 | 26 ± 5 | 13 ± 4 | 10 ± 3 | 3 ± 1** |
| 25-30 | 29 ± 5 | 14 ± 5 | 10 ± 3* | 3 ± 1** |
| 30-35 | 29 ± 5 | 15 ± 5 | 11 ± 3* | 4± 1** |
| 35-40 | 32 ± 5 | 15 ± 5* | 11 ± 3* | 4 ± 1** |
| 40-45 | 33 ± 6 | 17 ± 6 | 12 ± 3* | 4 ± 1** |
| 45-50 | 34 ± 6 | 18 ± 6 | 12 ± 3* | 4 ± 1*** |
| 50-55 | 36 ± 6 | 18 ± 6 | 12 ± 3* | 4 ± 1*** |
| 55-60 | 36 ± 6 | 18 ± 6 | 12 ± 3* | 4 ± 1*** |
| The data in the Table are mean value ± standard error. | | | | |

| | | | | |
|---|---|---|---|---|
| * p<0.05, | | | | |
| ** p<0.01, | | | | |
| *** p<0.001 (compared with control at each time), (n = 4, Dunnett's test). | | | | |

**Table 2**

| Time after Subcutaneous Injection (min) . | Control | Compound 2 (30 mg/kg, p.o.) |
|---|---|---|
| 0-10 | 17 ± 4 | 10 ± 2 |
| 10-20 | 22 ± 5 | 11 ± 3 |
| 20-30 | 24 ± 5 | 14 ± 2 |
| 30-40 | 27 ± 5 | 14 ± 2 |
| 40-50 | 30 ± 7 | 14 ± 2* |
| 50-60 | 31 7 | 14 ± 2* |
| The data in the Table are mean value ± standard error. | | |

| | | |
|---|---|---|
| * p<0.05 (compared with control at each time) , (n = 10, Student's t-test). | | |

The test data confirm that Compound 1 and Compound 2 inhibited the compound 48/80-induced scratching' in mice. Test Example 2: Inhibitory effect on poly-L-arginine-induced scratching in mice

The experiment was carried out according to the method of Hayashi, et al. [*Eur. J. Pharmacol*., Vol. 425, pp. 219-227 (2001)].

Male Wistar rats of 5 weeks (supplied by Japan SLC) were used in the test. 4 to 6 rats were put in each plastic cage and reared by allowing them to freely take commercially available solid feed andwater, in an animal roomat 19-25°C, 30-70% humidity and a 12 h lighting cycle (lighting: 7:00 a.m. to 7:00 p.m.) a day.

Every rat was shaven on the back, individually put in an acrylic monitor cage (200 x 145 x 170 mm) , and acclimated therein for 30 minutes. 50 µl of a solution of poly-L-arginine (by Sigma) (4 mg of poly-L-arginine was dissolved in 1 ml of physiological saline) was intracutaneously injected into the interscapular region of each rat. For 30 minutes immediately after this, the behavior of every rat was recorded with a 8-mm video camera recorder. The video was reproduced, and the number of the scratching by its back paws at around the area where the solution had been intracutaneously injected was counted for 30 minutes immediately after the intracutaneous administration. Each rat scratched itself a few times per about one second, and a series of these actions of each rat was counted as one.

Compound 1 was suspended in aqueous 0.5 w/v % methyl cellulose solution to have a concentration of 0.1 or 1 mg/ml. The 0.1 mg/ml suspension of Compound 1 was further diluted with aqueous 0.5 w/v % methyl cellulose solution, and suspensions or solutions for administration each having an intended concentration were prepared. The solvent (aqueous 0.5 w/v % methyl cellulose solution) or the suspension or solution for administration was orally administered to each rat, 2 hours before the intracutaneous injection of poly-L-arginine, and its volume was 10 ml/kg. The solvent-administered rats were a control group.

The data of Compound 1 (10 mg/kg, p.o.) to inhibit the poly-L-arginine-induced scratching in rats are shown in Table 3.

**Table 3**

| | Control | Compound 1 (10 mg/kg, p.o.) |
|---|---|---|
| Number of Scratching Actions for 30 minutes | 39 ± 10 | 14 ± 3* |
| The data in the Table are mean value ± standard error. | | |

| | | |
|---|---|---|
| * p<0.05 (compared with control), ( n = 8, Wilcoxon's rank-total test). | | |

The test data confirm that Compound 1 inhibited the poly-L-arginine-induced pruritic behavior of rats.

As in Test Example 1 and Test Example 2, Compound 1 inhibited the scratching of animals. Histamine participates in the compound 48/80-induced scratching in mice shown in Test Example 1 *[Eur. J. Pharmacol*., Vol. 351, pp. 1-5 (1998)], but does not in the poly-L-arginine-induced scratching in rats shown in Test Example 2 [*Eur. J. Pharmacol*., Vol. 425, pp. 219-227 (2001)]. Accordingly, it is shown that Compound 1 is effective not only for pruritus for which' antihistamines are effective but also even for pruritus for which antihistamines are ineffective. In addition, since Compound 2 inhibited the compound 48/80-induced scratching in mice, it is suggested that Compounds (I) and pharmaceutically acceptable salts thereof are useful as a preventive or therapeutic agent for pruritus. Test Example 3: Acute Toxicity Test

The test compound was administered orally or intraperitoneally to three/group male dd mice (body weight: 20 ± 1 g). The minimum lethal dose (MLD) was obtained by observing the mortality on the seventh day after the administration.

As a result, MLD of Compound 1 was >1000 mg/kg by oral administration.

Compounds (I) and pharmaceutically acceptable salts thereof can be used as such or in various pharmaceutical forms. The pharmaceutical compositions of the present invention can be produced by uniformly mixing an effective amount of Compound (I) or a pharmaceutically acceptable salt thereof, as an active ingredient, with a pharmaceutically acceptable carrier. It is preferred that these pharmaceutical compositions are in a unit dose form suitable for administration such as oral administration or parenteral administration (including intravenous administration).

In the preparation of compositions for oral administration, any useful pharmaceutically acceptable carriers can be used. For example, liquid preparations for oral administration such as suspensions and syrups can be produced using water, sugars such as sucrose, sorbitol and fructose, glycols such as polyethylene glycol and propylene glycol, oils such as sesame oil, olive oil and soybean oil, antiseptics such as p-hydroxybenzoates, flavors such as strawberry flavor and peppermint, or the like. Capsules, tablets, powders and granules can be produced using excipients such as lactose, glucose, sucrose and mannitol, disintegrators such as starch and sodium alginate, lubricants such as magnesium stearate and talc, binders such as polyvinyl alcohol, hydroxypropyl cellulose and gelatin, surfactants such as fatty acid esters, plasticizers such as glycerin, or the like. Tablets and capsules are the most useful unit dose forms for oral administration because of the easiness of administration. Solid pharmaceutical carriers are used for the production of tablets and capsules.

Injections can be prepared using, for example, carriers comprising distilled water, a salt solution, a glucose solution or a mixture of salt water and a glucose solution. They are prepared as solutions, suspensions or dispersions using appropriate auxiliaries according to conventional methods.

Compounds (I) or pharmaceutically acceptable salts thereof can be administered orally in the above pharmaceutical forms or parenterally as an injection or the like. The effective dose and administration schedule vary depending upon the mode of administration, the age, body weight and condition of a patient, or the like, but they are usually administered in a dose of 1 to 900 mg/60 kg/day, preferably 1 to 200 mg/60 kg/day.

Certain embodiments of the present invention are illustrated in the following examples.

### Best Modes for Carrying Out the Invention

### Example 1: Tablets

Tablets having the following composition were prepared according to a conventional method.

Compound 1 (250 g), mannitol (1598.5 g), sodium starch glycolate (100 g), light silicic acid anhydride (10 g), magnesium stearate (40 g) and yellow iron oxide (1.5 g) weremixed according to a conventional method. The resulting mixture was compressed using a tabletting machine equipped with 8 mm diameter punch and die (Purepress Correct-12, Kikusui Seisakusho Ltd.) to prepare tablets each containing 25 mg of the active ingredient.

The formulation is shown in Table 4.

**Table 4**

| Formulation | Compound 1 | 25 mg |
|---|---|---|
| | Mannitol | 159.85 mg |
| | Sodium starch glycolate | 10 mg |
| | Light silicic acid anhydride | 1 mg |
| | Magnesium stearate | 4 mg |
| | Yellow iron oxide | 0.15 mg |
| | | 200 mg |

### Example 2: Capsules

Capsules having the following composition were prepared according to a conventional method.

Compound 1 (500 g), lactose (300 g), light silicic acid anhydride (100 g) and sodium lauryl sulfate (100 g) were mixed according to a conventional method. The resulting mixture was encapsulated in hard capsules No. 1 (content: 100 mg/capsule) using a capsule filler (LZ-64, Zanasi) to prepare capsules each containing 50 mg of the active ingredient.

The formulation is shown in Table 5.

**Table 5**

| Formulation | Compound 1 | 50 mg |
|---|---|---|
| | Lactose | 30 mg |
| | Light silicic acid anhydride | 10 mg |
| | Sodium lauryl sulfate | 10 mg |
| | | 100 mg |

### Example 3: Injection

An injection having the following composition is prepared according to a conventional method.

Compound 1 (1 g) is dissolved in 100 g of purified soybean oil, and 12 g of purified egg yolk lecithin and 25 g of glycerin for injection are added thereto. The resulting mixture is made up to 1000 ml with distilled water for injection, kneaded and emulsified according to a conventional method. The obtained dispersion is aseptically filtered using a 0.2µm disposable membrane filter and aseptically packed in glass vials in 2 ml portions to prepare injections each containing 2 mg of the active ingredient per vial.

The formulation is shown in Table 6.

**Table 6**

| Formulation | Compound 1 | 2 mg |
|---|---|---|
| | Purified soybean oil | 200 mg |
| | Purified egg yolk lecithin | 24 mg |
| | Glycerin for injection | 50 mg |
| | Distilled water for injection | 1.72 ml |
| | | 2.00 ml |

### Industrial Applicability

The present invention provides a preventive or therapeutic agent for pruritus comprising, as an active ingredient, a tricyclic compound or a pharmaceutically acceptable salt thereof.

## Claims

1. A preventive or therapeutic agent for pruritus which comprises, as an active ingredient, a tricyclic compound represented by Formula (I): 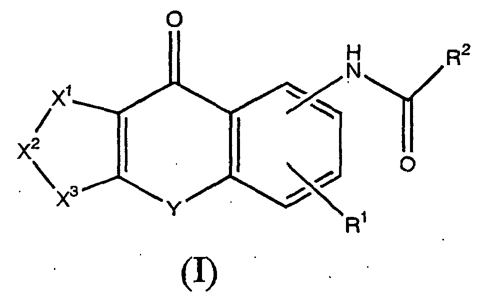 {wherein R¹ represents hydrogen, substituted or unsubstituted lower alkyl, substituted or unsubstituted lower alkoxy or halogen;
X¹-X²-X³ represents CR⁵=CR⁶-CR⁷=CR⁸ [wherein R⁵, R⁶, R⁷ and R⁸, which may be the same or different, each represent hydrogen, substituted or unsubstituted lower alkyl, hydroxy, substituted or unsubstituted lower alkoxy, nitro, amino, mono(lower alkyl)-substituted amino, di(lower alkyl)-substituted amino, substituted or unsubstituted lower alkanoylamino or halogen], N(O)ₘ=CR⁶-CR⁷=CR⁸ (wherein R⁶, R⁷ and R⁸ each have the same significances as defined above, and m represents 0 or 1), CR⁵=CR⁶-N(O)ₘ=CR⁸ (wherein R⁵, R⁶, R⁸ and m each have the same significances as defined above), CR⁵=CR⁶-CR⁷=N(O)ₘ (wherein R⁵, R⁶, R⁷ and m each have the same significances asdefined above), CR⁵=CR⁶-O (wherein R⁵ and R⁶ each have the same significances as defined above), CR⁵=CR⁶-S (wherein R⁵ and R⁶ each have the same significances as defined above), O-CR⁷=CR⁸ (wherein R⁷ and R⁸ each have the same significances as defined above), S-CR⁷=CR⁸ (wherein R⁷ and R⁸ each have the same significances as defined above) or O-CR⁷=N (wherein R⁷ has the same significance as defined above);
Y represents -CH₂S-, -CH₂SO-, -CH₂SO₂-, -CH₂O-, -CH=CH-, -(CH₂)ₚ- (wherein p represents an integer of 0 to 2), -SCH₂-, -SOCH₂-, -SO₂CH₂- or -OCH₂-; and
R² represents hydrogen, substituted or unsubstituted lower alkyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, or a substituted or unsubstituted heterocyclic group} or a pharmaceutically acceptable salt thereof.

2. A preventive or therapeutic agent for pruritus which comprises, as an active ingredient, a tricyclic compound represented by Formula (Ia): 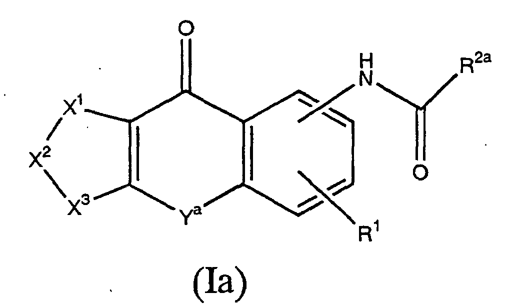 [wherein R¹ and X¹-X²-X³ each have the same significances as defined above; Y^{a} represents -CH₂SO₂-, -SCH₂-, -SOCH₂-, -SO₂CH₂- or -OCH₂-; and
when Y^{a} is -CH₂SO₂-, -SCH₂-, -SOCH₂- or -SO₂CH₂-,
R^{2a} represents hydrogen, substituted or unsubstituted lower alkyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, or a substituted or unsubstituted nitrogen-containing heterocyclic group; and
when Y^{a} is -OCH₂-,
R^{2a} represents hydrogen, trifluoromethyl, substituted or unsubstituted lower alkenyl, substituted or unsubstituted lower alkoxy, amino, mono(substituted or unsubstituted lower alkyl)-substituted amino, di(substituted or unsubstituted lower alkyl)-substituted amino, substituted or unsubstituted aryl, substituted or unsubstituted heteroaryl, substituted or unsubstituted aralkylamino, substituted or unsubstituted arylamino, a substituted or unsubstituted heteroalicyclic group, a substituted or unsubstituted nitrogen-containing heterocyclic group, or Formula (II): 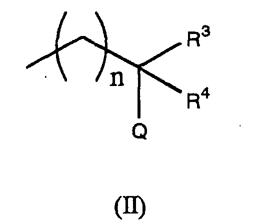 (wherein n is 0 or 1; R³ and R⁴, which may be the same or different, each represent hydrogen, substituted or unsubstituted lower alkyl, substituted or unsubstituted cycloalkyl, substituted or unsubstituted aryl, or substituted or unsubstituted aralkyl, or R³ and R⁴ may be combined together with the adjacent carbon atom to form cycloalkyl; and Q represents hydroxy, substituted or unsubstituted lower alkoxy, amino or halogen)] or a pharmaceutically acceptable salt thereof.

3. A preventive or therapeutic agent for pruritus which comprises, as an active ingredient, the tricyclic compound or the pharmaceutically acceptable salt thereof described in claim 2, wherein Y^{a} is -CH₂SO₂-, -SCH₂-, -SOCH₂- or -SO₂CH₂-.

4. A preventive or therapeutic agent for pruritus which comprises, as an active ingredient, the tricyclic compound or the pharmaceutically acceptable salt thereof described in claim 2, wherein Y^{a} is -OCH₂-.

5. A preventive or therapeutic agent for pruritus which comprises, as an active ingredient, the tricyclic compound or the pharmaceutically acceptable salt thereof described in any of claims 2 to 4, wherein R¹ is hydrogen, substituted or unsubstituted lower alkoxy or halogen.

6. A preventive or therapeutic agent for pruritus which comprises, as an active ingredient, the tricyclic compound or the pharmaceutically acceptable salt thereof described in any of claims 2 to 4, wherein R¹ is hydrogen.

7. A preventive or therapeutic agent for pruritus which comprises, as an active ingredient, the tricyclic compound or the pharmaceutically acceptable salt thereof described in any of claims 2, 5 and 6, wherein Y^{a} is -CH₂SO₂-, -SO₂CH₂- or -OCH₂-.

8. A preventive or therapeutic agent for pruritus which comprises, as an active ingredient, the tricyclic compound or the pharmaceutically acceptable salt thereof described in any of claims 2, 5 and 6, wherein Y^{a} is -CH₂SO₂- or -SO₂CH₂-.

9. A preventive or therapeutic agent for pruritus which comprises, as an active ingredient, the tricyclic compound or the pharmaceutically acceptable salt thereof described in any of claims 2, 5 and 6, wherein Y^{a} is -CH₂SO₂-.

10. A preventive or therapeutic agent for pruritus which comprises, as an active ingredient, the tricyclic compound or the pharmaceutically acceptable salt thereof described in any of claims 2 to 9, wherein X¹-X²-X³ is S-CR⁷=CR⁸ (wherein R⁷ and R⁸ each have the same significances as defined above).

11. A preventive or therapeutic agent for pruritus which comprises, as an active ingredient, the tricyclic compound or the pharmaceutically acceptable salt thereof described in any of claims 2 to 9, wherein X¹-X²-X³ is CR⁵=CR⁶-CR⁷=CR⁸ (wherein R⁵, R⁶, R⁷ and R⁸ each have the same significances as defined above).

12. A preventive or therapeutic agent for pruritus which comprises, as an active ingredient, the tricyclic compound or the pharmaceutically acceptable salt thereof described in any of claims 2 to 11, wherein R^{2a} is Formula (II): 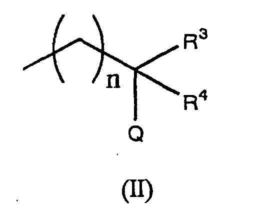 (wherein n, R³, R⁴ and Q each have the same significances as defined above).

13. A preventive or therapeutic agent for pruritus which comprises, as an active ingredient, the tricyclic compound or the pharmaceutically acceptable salt thereof described in claim 12, wherein n is 0.

14. A preventive or therapeutic agent for pruritus which comprises, as an active ingredient, the tricyclic compound or the pharmaceutically acceptable salt thereof described in claim 13, wherein R³ is methyl, R⁴ is trifluoromethyl, and Q is hydroxy.

15. A preventive or therapeutic agent for pruritus which comprises, as an active ingredient, the tricyclic compound or the pharmaceutically acceptable salt thereof described in claim 2, wherein R¹ is hydrogen, Y^{a} is -CH₂SO₂-, X¹-X²-X³ is S-CR⁷=CR⁸ (wherein R⁷ and R⁸ each have the same significances as defined above), and R² is Formula (III):

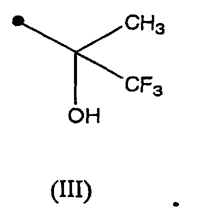

16. A preventive or therapeutic agent for pruritus which comprises, as an active ingredient, a tricyclic compound represented by Formula (Ib): 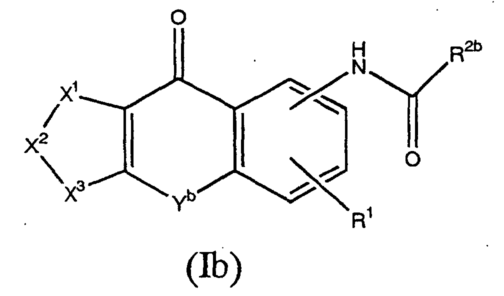 [wherein R¹ and X¹-X²-X³ each have the same significances as defined above;
Y^{b} represents -CH₂O-, -CH₂S-, -CH₂SO-, -CH=CH- or -(CH₂)ₚ- (wherein p has the same significance as defined above); and
R^{2b} represents Formula (III): 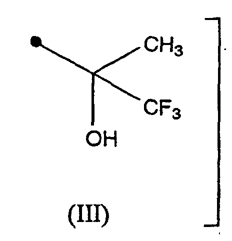 or a pharmaceutically acceptable salt thereof.

17. A preventive or therapeutic agent for pruritus which comprises, as an active ingredient, the tricyclic compound or the pharmaceutically acceptable salt thereof described in claim 16, wherein X¹-X²-X³ is CR⁵=CR⁶-CR⁷=CR⁸ (wherein R⁵, R⁶, R⁷ and R⁸ each have the same significances as defined above) or CR⁵=CR⁶-CR⁷=N (wherein R⁵, R⁶ and R⁷ each have the same significances as defined above).

18. A preventive or therapeutic agent for pruritus which comprises, as an active ingredient, the tricyclic compound or the pharmaceutically acceptable salt thereof described in claim 16, wherein X¹-X²-X³ is CR⁵=CR⁶-O (wherein R⁵ and R⁶ each have the same significances as defined above) or CR⁵=CR⁶-S (wherein R⁵ and R⁶ each have the same significances as defined above).

19. A preventive or therapeutic agent for pruritus which comprises, as an active ingredient, the tricyclic compound or the pharmaceutically acceptable salt thereof described in claim 16, wherein X¹-X²-X³ is O-CR⁷=CR⁸ (wherein R⁷ and R⁸ each have the same significances as defined above) or S-CR⁷=CR⁸ (wherein R⁷ and R⁸ each have the same significances as defined above).

20. A preventive or therapeutic agent for pruritus which comprises, as an active ingredient, the tricyclic compound or the pharmaceutically acceptable salt thereof described in any of claims 16 to 19, wherein Y^{b} is -CH₂O-.

21. A preventive or therapeutic agent for pruritus which comprises, as an active ingredient, the tricyclic compound or the pharmaceutically acceptable salt thereof described in any of claims 16 to 19, wherein Y^{b} is -(CH₂)ₚ- (wherein p has the same significance as defined above).

22. A preventive or therapeutic agent for pruritus which comprises, as an active ingredient, the tricyclic compound or the pharmaceutically acceptable salt thereof described in claim' 21, wherein p is 0.

23. A preventive or therapeutic agent for pruritus which comprises, as an active ingredient, the tricyclic compound or the pharmaceutically acceptable salt thereof described in claim 21, wherein p is 2.

24. A preventive or therapeutic agent for pruritus which comprises, as an active ingredient, the tricyclic compound or the pharmaceutically acceptable salt thereof described in any of claims 16 to 19, wherein Y^{b} is -CH=CH-.

25. A preventive or therapeutic agent for pruritus which comprises, as an active ingredient, the tricyclic compound or the pharmaceutically acceptable salt thereof described in any of claims 16 to 19, wherein Y^{b} is -CH₂S- or -CH₂SO-.

26. Use of the tricyclic compound or the pharmaceutically acceptable salt thereof described in any of claims 1 to 25 for the manufacture of a pharmaceutical composition useful for prevention or treatment of pruritus.

27. A method for preventing or treating pruritus, which comprises a step of administering the tricyclic compound or the pharmaceutically acceptable salt thereof described in any of claims 1 to 25.
